# EUROPEAN PATENT APPLICATION

(11) **EP 3 000 895 A1**
(43) Date of publication of application: **30.03.2016**
(21) Application number: 14306490.5
(22) Date of filing: 26.09.2014
(51) Int. Cl.: C12Q 1/68

(54) **A method for predicting responsiveness to a treatment with an EGFR inhibitor**

(71) Applicant: Integragen, 91000 Evry (FR)
(72) Inventor: Thiebaut, Raphaële, 78000 Versailles (FR)
(74) Representative: Regimbeau

(57) **Abstract**

The present invention relates to a method for predicting whether a patient with a cancer is likely to respond to an epidermal growth factor receptor (EGFR) inhibitor, which method comprises determining the expression level of hsa-miR-31-5p (SEQ ID NO:2) miRNA in a sample of said patient. The invention also relates to kits for measuring the expression of hsa-miR-31-5p and at least one other parameter positively or negatively correlated to response to EGFR inhibitors. The invention also relates to therapeutic uses of an EGFR inhibitor in a patient predicted to respond to said EGFR inhibitor.

## Description

### TECHNICAL FIELD OF THE INVENTION

The present invention provides methods for individualizing chemotherapy for cancer treatment, and particularly for evaluating a patient's responsiveness to one or more epidermal growth factor receptor (EGFR) inhibitors prior to treatment with such agents, based on the determination of the expression level of hsa-miR-31-5p.

### BACKGROUND OF THE INVENTION

The epidermal growth factor receptor (EGFR) pathway is crucial in the development and progression of human epithelial cancers. The combined treatment with EGFR inhibitors has a synergistic growth inhibitory and pro-apoptotic activity in different human cancer cells which possess a functional EGFR-dependent autocrine growth pathway through to a more efficient and sustained inhibition of Akt.

EGFR inhibitors have been approved or tested for treatment of a variety of cancers, including non-small cell lung cancer (NSCLC), head and neck cancer, colorectal carcinoma, and Her2-positive breast cancer, and are increasingly being added to standard therapy. EGFR inhibitors, which may target either the intracellular tyrosine kinase domain or the extracellular domain of the EGFR target, are generally plagued by low population response rates, leading to ineffective or non-optimal chemotherapy in many instances, as well as unnecessary drug toxicity and expense. For example, a reported clinical response rate for treatment of colorectal carcinoma with cetuximab (a chimeric monoclonal antibody targeting the extracellular domain of EGFR) is about 11% (Cunningham et al, N Engl Med 2004;351: 337-45), and a reported clinical response rate for treatment of NSCLC with erlotinib is about 8.9% (Shepherd F A, et al, N Engl J Med 2005; 353:123-132).

In particular resistance has been observed in case of *KRAS* mutation.

In colorectal cancer, as KRAS mutations are clearly associated with resistance to anti-EGFR antibodies (Lievre et al, Cancer Res. 2006 66(8):3992-5), one of the major challenges is to identify, in non-mutated KRAS patients, other markers that can predict lack of response to this therapy. Among them, amplification or activating mutations of oncogenes and inactivating mutations of tumor suppressor genes described above are relevant candidates, such as the level of activation of EGFR downstream signaling pathway evaluated by the measurement of EGFR downstream phosphoprotein expression.

In lung cancer, three groups of patients are emerging: one counts the patients with EGFR mutated tumors for which the use of EGFR tyrosine kinase inhibitors (EGFR TKI) was proven to improve outcome, the second counts the patients with KRAS mutated tumors for which anti-EGFR therapies are probably not the good alternatives, and the third group counts the non-EGFR and non-KRAS mutated tumors for which response cannot be predicted. No marker linked to drug response in the non-mutated tumor group has proved valuable so far.

Thus, there is a need for predicting patient responsiveness to EGFR inhibitors prior to treatment with such agents, so as to better individualize patent therapy.

There are many documents in the prior art concerning the involvement of micro RNAs (miRNAs) in sensitivity or resistance to various anticancer treatments. However, in most cases, studies are partial, incomplete, and actually do not permit a true prediction of clinical response or non-response to treatment. Indeed, in many cases, studies are limited to the analysis of the expression of miRNAs *in vitro,* in cell lines sensitive or resistant to a particular treatment, or in tumor cells isolated from a patient tumor. In addition, in many studies, while differences in expression level between two populations of cells or patients are shown, no threshold value or score actually permitting to predict response or non-response in a new patient are provided. This is partly linked to the first shortage that many studies lack data obtained in a clinical setting. Moreover, even when some data obtained in a clinical setting is presented, these data are most of the time only retrospective, and data validating a prediction method in a new cohort are often lacking.

As an example, WO2010/121238 describes the analysis of miRNAs expression in lung cancer cell lines sensitive or resistant to EGFR tyrosine kinase inhibitors cultures *in vitro.* No data obtained in a clinical setting is presented.

WO2009/080437 broadly claims methods for predicting response or non-response to anticancer treatment. However, data presented in WO2009/080437 is limited to various conventional chemotherapy treatments, and no data is provided concerning EGFR inhibitors (neither for anti-EGFR monoclonal antibodies nor for EGFR tyrosine kinase inhibitors). In addition, data presented for other chemotherapeutic molecules were obtained based on expression of miRNAs in tumor cells isolated from patient's tumors cultured in vitro. No data obtained in a clinical setting is presented.

Similarly, while WO2011/135459 broadly claims methods for predicting response or non-response to anticancer treatment, data presented in this document are limited to prediction of sensitivity or resistance of cancer cell lines to various anticancer agents in vitro. Here also, no data obtained in a clinical setting is presented, and thus no correlation between miRNA expression level and clinical response or survival of patient is demonstrated.

Ragusa et al-2010 (Ragusa M. et al. Mol Cancer Ther. 2010 Dec;9(12):3396-409) analyzed the expression level of miRNAs after treatment with cetuximab in colorectal cancer cell lines known to be sensitive or resistant to cetuximab treatment. Two miRNAs are shown to be differentially expressed in KRAS wild-type versus KRAS mutated patients. However, differential expression in KRAS wild-type versus KRAS mutated patients does not permit to predict response to EGFR inhibitors in KRAS wild-type patients. In addition, as in many other studies, no data obtained in a clinical setting showing the ability of the expression levels of these miRNAs to independently predict response to EGFR inhibitors in patients is presented.

WO 2013/076282 describes an *in vitro* method for predicting whether a patient with a cancer is likely to respond to an epidermal growth factor receptor (EGFR) inhibitor, which comprises determining the expression level of hsa-miR-31-3p (previously named hsa-miR-31*, UGCUAUGCCAACAUAUUGCCAU, accession number MIMAT0004504 on http://www.mirbase.org, SEQ ID NO:1 in the present description) miRNA in a sample of said patient. More particularly, the lower the expression of hsa-miR-31-3p is, the more likely the patient is to respond to the EGFR inhibitor treatment. While the method disclosed in this application is a true method for predicting whether a patient with a cancer is likely to respond to an epidermal growth factor receptor (EGFR) inhibitor, there is still a need for further true and validated methods for predicting response to EGFR inhibitors in patients for which such therapy is one of several options. The present invention provides a response to this need.

MicroRNAs (or miRNAs) are single-stranded molecule of about 21-24 nucleotides, preferably 21-23 in length, encoded by genes that are transcribed from DNA but not translated into protein (non-coding RNA); instead they are processed from primary transcripts known as *pri-miRNA* to short stem-loop structures called *pre-miRNA* and finally to functional miRNA. During maturation, each pre-miRNA gives rise to two distinct fragments with high complementarity, one originating from the 5' arm the other originating from the 3' arm of the gene encoding the pri-miRNA. The two mature miRNAs obtained either from the 5' or the 3' arm of the gene encoding the pri-miRNA are respectively referred to as the "5p" or "3p" miRNA. Most of the time, one of the two fragments (5p or 3p) is degraded and found in lesser amount than the other (3p or 5p) fragment (Liu N et al. Cell Res. 2008 Oct;18(10):985-96; Okamura K et al. Nat Struct Mol Biol. 2008 Apr;15(4):354-63). The most abundant fragment may then also be referred to as miR-X (X being the unique arbitrary number assigned to the sequence of the miRNA in the particular species), while the less abundant fragment may be referred to as miR-X*.

In WO 2013/076282, the miRNA used for predicting whether a patient with a cancer is likely to respond to an epidermal growth factor receptor (EGFR) inhibitor is hsa-miR-31-3p, also referred to as hsa-miR-31* (SEQ ID NO:1). The other fragment with high complementarity generated from the common pre-miRNA pre-miR-31 is hsa-miR-31-5p, also referred to as hsa-miR-31 (AGGCAAGAUGCUGGCAUAGCU, accession number MIMAT0000089 on http://www.mirbase.org SEQ ID NO:2).

Changes in hsa-miR-31-5p level of expression are known to be associated to diagnosis/prognosis of various cancers, although conflicting data have been obtained (Wang S et al. Tumour Biol. 2014 Aug 20; Laurila EM et al. Genes Chromosomes Cancer. 2013 Dec;52(12):1103-13).

hsa-miR-31-5p level of expression has also been shown to be associated to resistance to 5-fluorouracil -5-FU), radiation therapy, paclitaxel, docetaxel and cisplatin (Laurila EM et al. Genes Chromosomes Cancer. 2013 Dec;52(12):1103-13; Bhatnagar N et al. Cell Death Dis. 2010 Dec 9;1:e105). However, these publications have not analyzed the existence of a possible correlation between hsa-miR-31-5p level of expression and resistance or response to EGFR inhibitor treatment.

hsa-miR-31-5p is the most abundant fragment generated from the common pre-miRNA pre-miR-31, the less abundant fragment being hsa-miR-31-3p, which has been shown in WO 2013/076282 to be useful for predicting whether a patient with a cancer is likely to respond to an epidermal growth factor receptor (EGFR) inhibitor.

However, as explained above, for a particular pre-miRNA, while similar amounts of both fragments are generated from the common pre-miRNA, the mature miRNA/miRNA* ratio is asymmetric at steady-state, sometimes at a discrepancy of >10 000:1 (Liu N et al. Cell Res. 2008 Oct;18(10):985-96; Okamura K et al. Nat Struct Mol Biol. 2008 Apr;15(4):354-63).

In addition, it is known in the art that the expression level of a particular miRNA and the miRNA/miRNA* ratio may change spatially and temporally (Liu N et al. Cell Res. 2008 Oct;18(10):985-96; Okamura K et al. Nat Struct Mol Biol. 2008 Apr;15(4):354-63; Guo L et al. PLoS One. 2010 Jun 30;5(6):e11387). Therefore, the fact that the expression level of one of the miRNA fragments generated from a common pre-miRNA is correlated to response/resistance to a particular therapy does not imply that the other fragment with high complementarity generated from the same common pre-miRNA is also correlated to response/resistance to the same therapy.

For instance, WO2011/135459 discloses in Tables 1-129 miRNAs for which overexpression (Tables 1-65) or underexpression (Tables 65-129) is correlated with growth of tumor cell lines in the presence of various drugs, based on analysis using Affymetrix miRNA 1.0 arrays, which includes many couples of miRNAs obtained from the same precursor pre-miRNA. For tamoxifen treatment, miRNAs which overexpression was found correlated to response to tamoxifen are listed in Table 53, while miRNAs which underexpression was found correlated to response to tamoxifen are listed in Table 118. Analysis of Tables 53 and 118 shows that:
- 10 miRNAs couples (5p and 3p fragments obtained from the same pre-miRNA precursor) have a similar positive or negative correlation with response to tamoxifen:
   o correlation of overexpression: miR106b/ miR106b*, miR17/ miR17*, miR18a/ miR18a* et miR-93/ miR-93*;
   o correlation of underexpression: miR151-3p/ miR151-5p, miR193b/ miR193b*, miR22/miR22*, miR28-3p/ miR28-5p, miR30a/ miR30a*, miR99b/ miR99b*);
- For 17 miRNAs couples, over or under expression of only one of the two fragments has been found correlated to response to tamoxifen, the expression level of the other fragment obtained from the same pre-miRNA not being correlated to response to tamoxifen:
   o One fragment overexpression found correlated, the other fragment expression not correlated: miR106a, miR1228, miR195*, miR25*, miR34b, miR629*, miR671-5p, miR769-5p;
   o One fragment underexpression found correlated, the other fragment expression not correlated: miR10a, miR125a-5p, miR21, miR221, miR23a, miR23b, miR30e, miR34c-5p, miR424*;
- For one miRNA, miR149* overexpression is correlated to tamoxifen response, while miR149 underexpression is correlated to tamoxifen response; i.e. the expression levels of the two fragments obtained from the same pre-miRNA are inversely correlated to tamoxifen response.

The above example clearly illustrates that while in some cases (here 10/28 cases) the expression levels of both fragments obtained from the same miRNA precursor are similarly correlated to response to a drug, in most cases (here 18/28 cases), either only one of the fragments is correlated to response to a drug, or even the two fragments are inversely correlated to drug response. Therefore, the fact that the expression level of one of the miRNA fragments generated from a common pre-miRNA is correlated to response/resistance to a particular therapy clearly does not imply that the other fragment with high complementarity generated from the same common pre-miRNA is also correlated to response/resistance to the same therapy. Moreover, in the specific case of hsa-miR-31-5p (also referred to as hsa-miR-31) and hsa-miR-31-3p (also referred to as hsa-miR-31*), WO2011/135459 shows that only one of them, the other, or both, may be correlated to drug response, depending on the drug tested. In particular, Table 89 shows that underexpression of both hsa-miR-31 (i.e. hsa-miR-31-5p) and hsa-miR-31* (i.e. hsa-miR-31-3p) is correlated to response to melphalan. However, in response to cytoxan (see Tables 28 and 93) and fulverstrant (see Tables 61 and 126), only underexpression of hsa-miR-31 (i.e. hsa-miR-31-5p) is correlated to response. In contrast, in response to lomustine (see Tables 47 and 112), only underexpression of hsa-miR-31* (i.e. hsa-miR-31-3p) is correlated to response. This clearly illustrate that only one of hsa-miR-31-5p and hsa-miR-31-3p may be correlated to a drug response, and both miRNAs may even be inversely correlated to the same drug response.

In addition, genes known to be targeted by hsa-miR-31-5p and hsa-miR-31-3p are different, which rather suggests that both miRNAs are involved in distinct pathways, so that those skilled in the art would not have considered that hsa-miR-31-5p expression level might be correlated to likelihood of response to an epidermal growth factor receptor (EGFR) inhibitor. In particular, for each miRNA, target genes of this miRNA are listed in various databases and ranked from the most probable to the less probable target gene, based on several criteria, including experimental validation, sequence information. A search in miRNA.org database on September 22, 2014 for hsa-miR-31-5p and hsa-miR-31-3p target genes shows that the 12 most probable target genes of hsa-miR-31-5p and hsa-miR-31-3p are the following:

| Rank | hsa-miR-31-5p target genes | hsa-miR-31-3p target genes |
|---|---|---|
| 1 | ACAD8 | SCL30A5 |
| 2 | ZNF512 | MASP1 |
| 3 | HSD17B6 | GABBR2 |
| 4 | WSB2 | LRP1B |
| 5 | SH2D1A | BACH1 |
| 6 | RGAG1 | HAUS4 |
| 7 | SYNC | THBS2 |
| 8 | SFRS11 | EHBP1 |
| 9 | FNDC3A | TCEB1 |
| 10 | BEST3 | VPS13A |
| 11 | SMUG1 | POLR2K |
| 12 | CUL5 | ZMYM5 |

As clearly appears from above, there is no common target gene among the 12 most probable target genes of hsa-miR-31-5p and hsa-miR-31-3p, illustrating that both miRNAs have mainly distinct targets.

In addition, Mosakhani N et al. Cancer Genet. 2012 Nov;205(11):545-51 analyzed likelihood of response to anti-EGFR antibody therapy in metastatic colorectal cancer patients, using Agilent's miRNA Microarray System V2 (723 human and 76 human viral miRNAs, Sanger database v10.1 in http://microrna.sanger.ac.uk), which includes both hsa-miR-31-5p and hsa-miR-31-3p. However, only hsa-miR-31-3p (referred to as miR-31* in this article) - and not hsa-miR-31-5p - was found to be correlated to patient response to anti-EGFR therapy, thus suggesting that hsa-miR-31-5p would not be correlated to likelihood of response to anti-EGFR therapy in colorectal patients.

### SUMMARY OF THE INVENTION

However, based on quantitative PCR analysis of tumor samples of colorectal patients treated by anti-EGFR therapy, the inventors unexpectedly found that hsa-miR-31-5p expression level is in fact also correlated to likelihood of response to an epidermal growth factor receptor (EGFR) inhibitor and may be used for predicting whether a patient with a cancer is likely to respond to an epidermal growth factor receptor (EGFR) inhibitor.

Based on the results obtained by the inventors (see Example 1), the present invention provides an *in vitro* method for predicting whether a patient with a cancer is likely to respond to an epidermal growth factor receptor (EGFR) inhibitor, which comprises determining the expression level of hsa-miR-31-5p (SEQ ID NO:2) miRNA in a sample of said patient.

Preferably the patient has a KRAS wild-type cancer.

The cancer preferably is a colorectal cancer, preferably a metastatic colorectal cancer.

In a most preferred embodiment, the invention provides an *in vitro* method for predicting whether a patient with a metastatic colorectal carcinoma is likely to respond to an epidermal growth factor receptor (EGFR) inhibitor, in particular an anti-EGFR antibody such as cetuximab or panitumumab, which method comprises determining the expression level of hsa-miR-31-5p (SEQ ID NO:2) miRNA in a tumor sample of said patient.

The invention also provides a kit for determining whether a patient with a cancer is likely to respond to an epidermal growth factor receptor (EGFR) inhibitor, comprising or consisting of: reagents for determining the expression level of hsa-miR-31-5p (SEQ ID NO:2) miRNA in a sample of said patient, and reagents for determining at least one other parameter positively or negatively correlated to response to EGFR inhibitors.

The invention further relates to an EGFR inhibitor for use in treating a patient affected with a cancer, wherein the patient has been classified as being likely to respond, by the method according to the invention.

The invention also relates to the use of an EGFR inhibitor for the preparation of a drug intended for use in the treatment of cancer in patients that have been classified as "responder" by the method of the invention.

The invention also relates to a method for treating a patient affected with a cancer, which method comprises (i) determining whether the patient is likely to respond to an EGFR inhibitor, by the method of the invention, and (ii) administering an EGFR inhibitor to said patient if the patient has been determined to be likely to respond to the EGFR inhibitor.

### BRIEF DESCRIPTION OF THE FIGURES

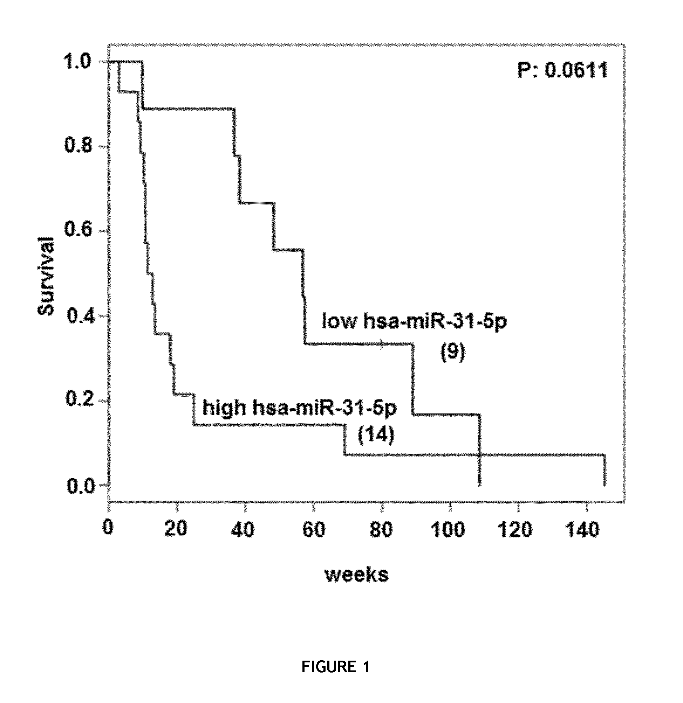

**Figure 1****:** survival curves (Kaplan-Meier) in patients depending on hsa-miR-31-5p expression level. Survival (expressed as the ratio of alive patients to all patients of the group of interest) is presented in function of time (weeks). The number of total patients in each group (low/high hsa-miR-31-5p) is mentioned in parentheses.

### DETAILED DESCRIPTION OF THE INVENTION

### Definitions

The **"patient"** may be any mammal, preferably a human being, whatever its age or sex. The patient is afflicted with a cancer. The patient may be already subjected to a treatment, by any chemotherapeutic agent, or may be untreated yet.

The cancer is preferably a cancer in which the signaling pathway through EGFR is involved. In particular, it may be *e.g.* colorectal, lung, breast, ovarian, endometrial, thyroid, nasopharynx, prostate, head and neck, kidney, pancreas, bladder, or brain cancer (Ciardello F et al. N Engl J Med. 2008 Mar 13;358(11):1160-74; Wheeler DL et al. Nat RevClinOncol. 2010 September ; 7(9): 493-507 ; Zeineldin R et al. J Oncol. 2010;2010:414676;Albitar L et al. Mol Cancer 2010;9:166; Leslie KK et al. GynecolOncol. 2012 Nov;127(2):345-50; Mimeault M et al. PLoS One.2012;7(2):e31919; Liebner DA et al. TherAdvEndocrinolMetab. 2011 Oct;2(5): 173-95; Leboulleux S et al. Lancet Oncol. 2012 Sep;13(9):897-905; Pan J et al. Head Neck. 2012 Sep 13; Chan SL et al. Expert OpinTher Targets. 2012 Mar;16 Suppl 1:S63-8; Chu H et al. Mutagenesis.2012 Oct 15; Li Y et al. Oncol Rep. 2010 Oct;24(4):1019-28; Thomasson M et al. Br J Cancer 2003, 89:1285-1289; Thomasson M et al. BMC Res Notes.2012 May 3;5:216). In certain embodiments, the tumor is a solid tissue tumor and/or is epithelial in nature. For example, the patient may be a colorectal carcinoma patient, a Her2-positive or Her2-negative (in particular triple negative, i.e. Her2-negative, estrogen receptor negative and progesterone receptor negative) breast cancer patient, a non-small cell lung cancer (NSCLC) patient, a head and neck cancer patient (in particular a squamous-cell carcinoma of the head and neck patient), a pancreatic cancer patient, or an endometrial cancer patient. More particularly, the patient may be a colorectal carcinoma patient, a Her2-positive or Her2-negative (in particular triple negative) breast cancer patient, a lung cancer (in particular a NSCLC) patient, a head and neck cancer patient (in particular a squamous-cell carcinoma of the head and neck patient), or a pancreatic cancer patient.

In a preferred embodiment, the cancer is a colorectal cancer, still preferably the cancer is a metastatic colorectal cancer. Indeed, data presented in Example 1 clearly indicate that hsa-miR-31-5p expression level may be used as a predictor of response to EGFR inhibitors (and in particular to anti-EGFR monoclonal antibodies such as cetuximab and panitumumab) treatment in colorectal cancer.

These results, obtained in a cancer in which the EGFR signaling pathway is known to be involved, clearly suggest that hsa-miR-31-5p expression level might be used as a predictor of response to EGFR inhibitors (and in particular to anti-EGFR monoclonal antibodies such as cetuximab and panitumumab) in any other cancer in which the EGFR signaling pathway is known to be involved, such as lung, ovarian, endometrial, thyroid, nasopharynx, prostate, head and neck, kidney, pancreas, bladder, or brain cancer.

Therefore, in another preferred embodiment, the cancer is a Her2-positive or Her2-negative (in particular triple negative) breast cancer, preferably a Her2-negative (in particular triple negative) breast cancer.

In still another preferred embodiment, the cancer is a lung cancer, in particular a non-small cell lung cancer (NSCLC).

In still another preferred embodiment, the cancer is a pancreatic cancer.

Since the prediction relates to EGFR inhibitors treatment, the patient's tumor is preferably EGFR positive.

Preferably, the patient has a KRAS wild-type tumor, *i.e.,* the KRAS gene in the tumor of the patient is not mutated in codon 12, 13 (exon 1), or 61 (exon 3). In other words, the KRAS gene is wild-type on codons 12, 13 and 61.

Wild type, i.e. non mutated, codons 12, 13 (exon 1), and 61 (exon 3) respectively correspond to glycine (Gly, codon 12), glycine (Gly, codon 13), and glutamine (Gln, codon 61). The wild-type reference KRAS amino acid sequence may be found in Genbank accession number NP_004976.2 (SEQ ID NO:3).

Especially the KRAS gene of the patient's tumor does not show any of the following mutations (Bos. Cancer Res 1989;49:4682-4689; Edkins et al. Cancer BiolTher. 2006 August ; 5(8): 928-932; Demiralay et al. Surgical Science, 2012, 3, 111-115):
Gly12Ser (GGT>AGT)
Gly12Arg (GGT>CGT)
Gly12Cys (GGT>TGT)
Gly12Asp (GGT>GAT)
Gly12Ala (GGT>GCT)
Gly12Val (GGT>GTT)
Gly13Arg (GGC>CGC)
Gly13Cys (GGC>TGC)
Gly13Asp (GGC>GAC)
Gly13Ala (GGC>GCC)
Gly13Val (GGC>GTC)

Preferably, the KRAS gene of the patient's tumor does also not show any of the following mutations (Demiralay et al. Surgical Science, 2012, 3, 111-115):
Gly12Phe (GGT>TTT)
Gly13Ser (GGC>AGC)

Preferably, the KRAS gene of the patient's tumor does also not show any of the following mutations (Bos. Cancer Res 1989;49:4682-4689; Tam et al. Clin Cancer Res2006;12:1647-1653 ; Edkins et al. Cancer BiolTher. 2006 August ; 5(8): 928-932; Demiralay et al. Surgical Science, 2012, 3, 111-115):
Gln61 His (CAA>CAC)
Gln61 His (CAA>CAT)
Gln61Arg (CAA>CGA)
Gln61 Leu (CAA>CTA)
Gln61Glu (CAA>GAA)
Gln61Lys (CAA>AAA)
Gln61Pro (CAA>CCA)

Any method known in the art may be used to know the KRAS status of the patient.

For example, a tumor tissue is microdissected and DNA extracted from paraffin-embedded tissue blocks. Regions covering codons 12, 13, and 61 of the KRAS gene are amplified using polymerase chain reaction (PCR). Mutation status is determined by allelic discrimination using PCR probes (Laurent-Puig P, et al, J ClinOncol. 2009, 27(35):5924-30) or by any other methods such as pyrosequencing (Ogino S, et al. J MolDiagn 2008;7:413-21).

The **"sample"** may be any biological sample derived from a patient, which contains nucleic acids. Examples of such samples include fluids (including blood, plasma, saliva, urine, seminal fluid), tissues, cell samples, organs, biopsies, etc. Preferably the sample is a tumor sample, preferably a tumor tissue biopsy or whole or part of a tumor surgical resection. The sample may be collected according to conventional techniques and used directly for diagnosis or stored. A tumor sample may be fresh, frozen or paraffin-embedded. Usually, available tumor samples are frozen or paraffin-embedded, most of the time paraffin-embedded.

By a **"reference sample",** it is meant a tumor sample (notably a tumor biopsy or whole or part of a tumor surgical resection) of a patient whose positive or negative response to an EGFR inhibitor treatment is known. Preferably, a pool of reference samples comprises at least one (preferably several, more preferably at least 5, more preferably at least 6, at least 7, at least 8, at least 9, at least 10) responder patient(s) and at least one (preferably several, more preferably at least 6, at least 7, at least 8, at least 9, at least 10) non-responder patient(s). The highest the number of responders (also referred to as "positive") and non-responders (also referred to as "negative") reference samples, the better for the reliability of the method of prediction according to the invention.

Within the context of this invention, a patient who is **"likely to respond"** or is **"responder"** refers to a patient who may respond to a treatment with an EGFR inhibitor, i.e. at least one of his symptoms is expected to be alleviated, or the development of the disease is stopped, or slowed down. Complete responders, partial responders, or stable patients according to the RECIST criteria (Eisenhauer et al, European Journal of Cancer, 2009, 45:228-247) are considered as "likely to respond" or "responder" in the context of the present invention.

In solid tumors, the RECIST criteria are an international standard based on the presence of at least one measurable lesion. "Complete response" means disappearance of all target lesions; "partial response" means 30% decrease in the sum of the longest diameter of target lesions, "progressive disease" means 20% increase in the sum of the longest diameter of target lesions, "stable disease" means changes that do not meet above criteria.

The term **"predicting"** or **"prognosis"** refers to a probability or likelihood for a patient to respond to the treatment with an EGFR inhibitor.

According to the invention, the sensitivity of tumor cell growth to inhibition by an EGFR inhibitor is predicted by whether and to which level such tumor cells express hsa-miR-31-5p.

The term **"treating"** or **"treatment"** means stabilizing, alleviating, curing, or reducing the progression of the cancer.

A **"miRNA"** or **"microRNA"** is a single-stranded molecule of about 21-24 nucleotides, preferably 21-23 in length, encoded by genes that are transcribed from DNA but not translated into protein (non-coding RNA); instead they are processed from primary transcripts known as *pri-miRNA* to short stem-loop structures called *pre-miRNA* and finally to functional miRNA. During maturation, each pre-miRNA gives rise to two distinct fragments with high complementarity, one originating from the 5' arm the other originating from the 3' arm of the gene encoding the pri-miRNA. Mature miRNA molecules are partially complementary to one or more messenger RNA (mRNA) molecules, and their main function is to downregulate gene expression.

There is an international nomenclature of miRNAs (see Ambros V et al, RNA 2003 9(3):277-279 ; Griffiths-Jones S. NAR 2004 32(Database Issue):D109-D111; Griffiths-Jones S et al. NAR 2006 34(Database Issue):D140-D144; Griffiths-Jones S et al. NAR 2008 36(Database Issue):D154-D158; and Kozomara A et al. NAR 2011 39(Database Issue):D152-D157), which is available from miRBase at http://www.mirbase.org/. Each miRNA is assigned a unique name with a predefined format, as follows:

For a mature miRNA: sss-miR-X-Y, wherein
- "sss" is a three letters code indicating the species of the miRNA, "hsa" standing for human,
- the upper case "R" in miR indicates that it is referred to a mature miRNA. However, some authors in the literature abusively use "mir" also for mature miRNA. In this case, it may be recognized that it is referred to a mature miRNA by the presence of "-Y",
- X is the unique arbitrary number assigned to the sequence of the miRNA in the particular species, which may be followed by a letter if several highly homologous miRNAs are known. For instance, "20a" and "20b" refer to highly homologous miRNAs.
- Y indicates whether the mature miRNA, which has been obtained by cutting of the pre-miRNA, corresponds to the 5' arm (Y is then "5p") or 3' arm (Y is then "3p") of the gene encoding the pri-mRNA. In previous international nomenclature of miRNAs, "-Y" was not present. The two mature miRNAs obtained either from the 5' or the 3' arm of the gene encoding the pri-miRNA were then distinguished by the presence or absence of a "*" sign just after n. The presence of the "*" sign indicated that the sequence corresponded to the less often detected miRNA. Since such classification was subject to changes, a new nomenclature using the "3p" and "5p" code has been implemented.

For a pri-miRNA:sss-mir-X, wherein
- sss is a three letters code indicating the species of the miRNA, "hsa" standing for human,
- the lower case "r" in mir indicates that it is referred to a pri-miRNA and not to a mature miRNA, which is confirmed by the absence of "-Y",
- n is the unique arbitrary number assigned to the sequence of the miRNA in the particular species, which may be followed by a letter if several highly homologous miRNAs are known.

Each miRNA is also assigned an accession number for its sequence.

The miRNA detected in the present invention is hsa-miR-31-5p (previously named hsa-miR-31). In this name, "hsa" means that it relates to a human miRNA, "miR" refers to a mature miRNA, "31" refers to the arbitrary number assigned to this particular miRNA, and "5p" means that the mature miRNAs has been obtained from the 5' arm of the gene encoding the pri-miRNA.
hsa-miR-31-5p is AGGCAAGAUGCUGGCAUAGCU (SEQ ID NO : 2)
(Accession number MIMAT0000089 on http://www.mirbase.org)

### Methods of Measuring hsa-miR-31-5p Expression Levels in a Sample

The expression level of the miRNA may be determined, e.g. the miRNAs may be quantified, by any method known by anyone skilled in the art.

Such measures are made in vitro, starting from a patient's sample, in particular a tumor sample, and necessary involve transformation of the sample. Indeed, no measure of a specific gene expression level can be made without some type of transformation of the sample.

Most technologies rely on the use of reagents specifically binding to the miRNA of interest, thus resulting in a modified sample further including the detection reagent. In addition, most technologies also involve some preliminary extraction of RNA from the patient's sample before binding to a specific reagent. The claimed method may thus also comprise a preliminary step of extracting RNA from the patient's sample.

Detection by mass spectrometry does not necessary involve preliminary binding to specific reagents. However, it is most of the time performed on extracted RNA. Even when performed directly on the sample, without preliminary extraction steps, it involves some extraction of molecules from the sample by the laser beam, which extracted molecules are then analysed by the spectrometer.

In any case, no matter which technology is used, the state of the sample after measure of a miRNA expression level has been transformed compared to the initial sample taken from the patient.

The amount of miRNA can be measured by any technology known by a person skilled in the art, including miRNA microarrays, quantitative PCR, next generation sequencing and hybridization with a labelled probe, including the nanostring technology (see Geiss GK et al. Nat Biotechnol. 2008 Mar;26(3):317-25).

In particular, real time quantitative RT-PCR (qRT-PCR) may be useful. In some embodiments, qRT-PCR can be used for both the detection and quantification of RNA targets (Bustin et al., 2005, Clin. Sci., 109:365-379). Quantitative results obtained by qRT-PCR can sometimes be more informative than qualitative data, and can simplify assay standardization and quality management. Thus, in some embodiments, qRT-PCR-based assays can be useful to measure hsa-miR-31-5p expression levels during cell-based assays. The qRT-PCR method may be also useful in monitoring patient therapy. qRT-PCR is a well-known and easily available technology for those skilled in the art and does not need a precise description. Examples of qRT-PCR-based methods can be found, for example, in U.S. Pat. No. 7,101,663. Commercially available qRT-PCR based methods (e.g.,Taqman® Array) may for instance be employed, the design of primers and/or probe being easily made based on the sequence of hsa-miR-31-5p disclosed above.

miRNA assays or arrays can also be used to assess the levels of the miRNAs in a sample.

In some embodiments, n miRNA oligonucleotide array can be prepared or purchased. An array typically contains a solid support and at least one oligonucleotide contacting the support, where the oligonucleotide corresponds to at least a portion of a miRNA.

Any suitable assay platform can be used to determine the presence of the miRNA in a sample. For example, an assay may be in the form of a membrane, a chip, a disk, a test strip, a filter, a microsphere, a multiwell plate, and the like. An assay system may have a solid support on which an oligonucleotide corresponding to the miRNA is attached. The solid support may comprise, for example, a plastic, silicon, a metal, a resin, or a glass. The assay components can be prepared and packaged together as a kit for detecting an miRNA. To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the person skilled in the art.

In another embodiment, the miRNA quantification may be performed using nanostring technology, as described in Geiss GK et al. Nat Biotechnol. 2008 Mar;26(3):317-25).

In another embodiment, the miRNA quantification may be performed by sequencing.

### Classifying the patient

### Classification based on hsa-miR-31-5p expression level(s)

The lower the expression of hsa-miR-31-5p is, the better for the patient. Therefore, the lower the level of expression of hsa-miR-31-5p is, the more likely the patient is to respond to the EGFR inhibitor treatment. In a preferred embodiment, the patient may thus be predicted as likely or unlikely to respond to an EGFR inhibitor (in particular an anti-EGFR antibody such a cetuximab or panitumumab) based on comparison of the hsa-miR-31-5p expression level in the patient's sample (in particular a tumor sample as described above) with one or more threshold value(s).

In a particular embodiment, the patient is considered as "responder", or likely to respond to a treatment with an EGFR inhibitor, when the expression level of hsa-miR-31-5p is lower than a threshold value. Such a threshold value may be determined based on a pool of reference samples, as defined above. In this embodiment, patients are classified into two groups based on hsa-miR-31-5p expression level, depending if this expression level is lower or greater than said threshold value. Patients with a hsa-miR-31-5p expression level lower than the threshold value are considered as likely to respond, i.e. as responders. In contrast, patients with a hsa-miR-31-5p expression level greater than or equal to the threshold value are considered as unlikely to respond, i.e. as non-responders.

In other particular embodiments, the method may be performed with several threshold values. In this case, patients are classified into at least three groups associated to distinct probabilities of response based on hsa-miR-31-5p expression level.

In the case of three groups, two threshold values are used, and patients are classified into three groups depending if their hsa-miR-31-5p expression level is low (i.e. lower than a first threshold value), intermediate (i.e. greater than or equal to the first threshold value and lower than a second threshold value), or high (i.e. greater than or equal to the second threshold value). Then, in a preferred embodiment, patients in the low expression group may then be considered as likely to respond, i.e. as responders (high probability of response), patients in the high expression group as unlikely to respond, i.e. as non-responders (low probability of response), and patients in the intermediate expression group are considered as having a moderate probability of response. Alternatively, a low, moderate of high probability of response may be given to the clinician, who may then decide whether or not to administer the EGFR inhibitor treatment.

In another embodiment, the method further comprises determining a prognostic score or index based on the expression level of hsa-miR-31-5p, wherein the prognostic score indicates whether the patient is likely to respond to the EGFR inhibitor. In particular, said prognosis score may indicate whether the patient is likely to respond to the EGFR inhibitor depending if it is higher or lower than a predetermined threshold value (dichotomized result). In another embodiment, a discrete probability of response or non-response to the EGFR inhibitor may be derived from the prognosis score.

The probability that a patient responds to an EGFR inhibitor treatment is linked to the probability that this patient survives, with or without disease progression, if the EGFR inhibitor treatment is administered to said patient.

As a result, a prognosis score may be determined based on the analysis of the correlation between the expression level of hsa-miR-31-5p and progression free survival (PFS) or overall survival (OS) of a pool of reference samples, as defined above. A PFS and/or OS score, which is a function correlating PFS or OS to the expression level of hsa-miR-31-5p, may thus be used as prognosis score for prediction of response to an EGFR inhibitor. Preferably, a PFS score is used, since absence of disease progression is a clear indicator of response to the EGFR inhibitor treatment.

Experimental data obtained by the inventors shows that the probability for a patient to respond to an EGFR inhibitor treatment is linearly and negatively correlated to the logged expression level of hsa-miR-31-5p (see **Figure 1**). In a preferred embodiment, said prognosis score is thus represented by the following formula:
Prognosis score = a * x + b, wherein x is the logged expression level of hsa-miR-31-5p (preferably log in base 2, referred to as "log₂") measured in the patient's sample, and a and b are parameters that have been previously determined based on a pool of reference samples, as defined above.

Depending if a is positive/negative, the patient may then be predicted as responding to the EGFR inhibitor if his/her prognosis score is greater than or equal to/lower than or equal to a threshold value c, and not responding to the EGFR inhibitor if his/her prognosis score is lower than/greater than threshold value c, wherein the value of c has also been determined based on the same pool of reference samples:
If a is positive, the patient may then be predicted as responding to the EGFR inhibitor if his/her prognosis score is greater than or equal to threshold value c, and not responding to the EGFR inhibitor if his/her prognosis score is lower than threshold value c.

Alternatively, if a is negative, then the patient may be predicted as responding to the EGFR inhibitor if his/her prognosis score is lower than or equal to threshold value c, and not responding to the EGFR inhibitor if his/her prognosis score is greater than threshold value c.

Based on the experiments performed by the inventors, it has been determined that, a, b and c are preferably in the following ranges:
- a : [0.096], preferably [0.001; 0.12];
- b : [0.144], preferably [0.01; 0.3];and
- c : [-0.1; 0.1], preferably [-0.055; 0.055].

In another embodiment, a discrete probability of response or non-response to the EGFR inhibitor may be derived from the above a * x + b prognosis score. A precise correlation between the prognosis score and the probability of response to the EGFR inhibitor treatment may be determined based on the same set of reference samples. Depending if a is positive/negative, a higher/lower prognosis score indicates a higher probability of response to the EGFR inhibitor treatment:
If a is positive, the higher the prognosis score, the higher is the probability of response to the EGFR inhibitor treatment (i.e. the lower is the probability of disease progression in the case of a PFS score).

Alternatively, if a is negative, then the lower the prognosis score, the higher is the probability of response to the EGFR inhibitor treatment (i.e. the lower is the probability of disease progression in the case of a PFS score).

This prediction of whether a patient with a cancer is likely to respond to an EGFR inhibitor may also be made using a nomogram. In a nomogram, points scales are established for each variable of a score of interest. For a given patient, points are allocated to each of the variables by selecting the corresponding points from the points scale of each variable. For a discrete variable (such as a gene expression level), the number of points attributed to a variable is linearly correlated to the value of the variable. For a dichotomized variable (only two values possible), two distinct values are attributed to each of the two possible values or the variable. The score of interest is then calculated by adding the points allocated for each variable (total points). Based on the value of the score, the patient may then be given either a good or bad response prognosis depending on whether the composite score is inferior or superior to a threshold value (dichotomized score), or a probability of response or non-response to the treatment.

It is clear that nomograms are mainly useful when several distinct variables are combined in a composite score (see below the possibility to use composite scores combining hsa-miR-31-5p expression level and DBNDD2 and/or EPB41L4B expression level(s); hsa-miR-31-5p expression level and hsa-miR-31-3p expression level; or hsa-miR-31-5p expression level and BRAF status). However, a nomogram may also be used to represent a prognosis score based on only one variable, such as hsa-miR-31-5p expression level. In this case, total points correspond to points allocated to the single variable.

Therefore, in an embodiment of the method for predicting whether a patient with a cancer is likely to respond to an EGFR inhibitor according to the invention, the method further comprises determining a risk of non-response based on a nomogram calibrated based on a pool of reference samples. The nomogram may be calibrated based on OS or PFS data. If calibrated based on OS, the risk of non-response corresponds to a risk of death. If calibrated based on PFS, the risk of non-response corresponds to a risk of disease progression.

### Classification based on hsa-miR-31-5p expression level and further parameters positively or negatively correlated to response to EGFR inhibitors

While response to EGFR inhibitors can be predicted based only on the expression level of hsa-miR-31-5p (see **Example 1**), the method according to the invention may also comprise determining at least one other parameter positively or negatively correlated to response to EGFR inhibitors.

In this case, a composite score combining the expression level of hsa-miR-31-5p and the other parameter(s) may notably be created based on a pool of reference samples.

A nomogram, in which points scales are established for each variable of the composite score, may also be used to combine the expression level of hsa-miR-31-5p and the other parameter(s), and obtain the composite score, which may then be correlated to the risk of non-response (i.e. the risk of disease progression for a PFS score). For a given patient, points are allocated to each of the variables by selecting the corresponding points from the points scale of each variable. For a discrete variable (such as hsa-miR-31-5p expression level or age), the number of points attributed to a variable is linearly correlated to the value of the variable. For a dichotomized variable (only two values possible, such as BRAF mutation status or gender), two distinct values are attributed to each of the two possible values or the variable.

A composite score is then calculated by adding the points allocated for each variable (total points). Based on the value of the composite score, the patient may then be given either a good or bad response prognosis depending on whether the composite score is inferior or superior to a threshold value (dichotomized score), or a probability of response or non-response to the treatment.

The points scale of each variable, as well the threshold value over/under which the response prognosis is good or bad or the correlation between the composite score and the probability of response or non-response may be determined based on the same pool of reference samples.

Such other parameters positively or negatively correlated to response to EGFR inhibitors may notably be selected from:
- age;
- gender;
- the expression level of hsa-miR-31-3p, which may be measured by any method disclosed above for measuring the expression level of hsa-miR-31-5p; and/or
- the presence or absence of at least one mutation positively or negatively correlated to response to EGFR inhibitors.

Such mutations may be detected by any method known to those skilled in the art and notably include those mentioned in **Table 1** below:

| Gene symbol | Unigene number | Chromosome | Genbank reference wild-type protein sequence(s) | Mutation* |
|---|---|---|---|---|
| Kras | Hs.505033 | 12 | NP_004976.2 (SEQ ID NO :3) | G12 |
| | | | | G13 |
| | | | | Q61 |
| | | | | K117N |
| | | | | A146 |
| BRAF | Hs.550061 | 7 | NP_004324.2 (SEQ ID NO:4) | V600 |
| NRAS | Hs.486502 | 1 | NP_002515.1 (SEQ ID NO:5) | G12 |
| | | | | G13 |
| | | | | Q61 |
| | | | | K117 |
| | | | | A146T |
| PIK3CA | Hs.553498 | 3 | NP_006209.2 (SEQ ID NO:6) | E545 |
| | | | | H1047 |
| EGFR | Hs.488293 | 7 | NP_005219.2 (SEQ ID NO:7) ; NP_958441.1 (SEQ ID NO:8) ; NP_958439.1 (SEQ ID NO:9) ; | S492R |
| AKT1 | Hs.525622 | 14 | NP_001014431.1 (SEQ ID NO: 10) ; NP_001014432.1 (SEQ ID NO: 11) ; NP_005154.2 (SEQ ID NO:12) | E17K |

| | | | | |
|---|---|---|---|---|
| * Mutations are defined by mention of the codon number in the protein, preceded by the one letter code for the wild-type amino acid, and optionally followed by the replacement amino acid. When no replacement amino acid is mentioned, the replacement amino acid may be any amino acid different from the wild-type amino acid. | | | | |

### EGFR Inhibitors

The present invention makes it possible to predict a patient's responsiveness to one or more epidermal growth factor receptor (EGFR) inhibitors prior to treatment with such agents.

The EGFR inhibitor may be an EGFR tyrosine kinase inhibitor, or may alternatively target the extracellular domain of the EGFR target. In certain embodiments, the EGFR inhibitor is a tyrosine kinase inhibitor such as Erlotinib, Gefitinib, or Lapatinib, or a molecule that targets the EGFR extracellular domain such as Cetuximab or Panitumumab.

Preferably the EGFR inhibitor is an anti-EGFR antibody, preferably a monoclonal antibody, in particular Cetuximab or Panitumumab.

Molecules that target the EGFR extracellular domain, including anti-EGFR monoclonal antibodies such as Cetuximab or Panitumumab, are mainly used in the treatment of colorectal cancer or breast cancer treatment. As a result, if the patient's cancer is colorectal cancer (in particular metastatic colorectal cancer) or breast cancer, then the method according to the invention may preferably be used to predict response to molecules that target the EGFR extracellular domain, and in particular to anti-EGFR monoclonal antibodies, such as Cetuximab or Panitumumab.

Conversely, tyrosine kinase EGFR inhibitors are mainly used in the treatment of lung cancer (in particular non-small cell lung cancer, NSCLC), so that if the patient's cancer is lung cancer (in particular non-small cell lung cancer, NSCLC), then the method according to the invention may preferably be used to predict response to tyrosine kinase EGFR inhibitors, such as Erlotinib, Gefitinib, or Lapatinib.

In pancreatic cancer or head and neck cancer (in particular squamous cell carcinoma of the head and neck (SCCHN)), both tyrosine kinase EGFR inhibitors and anti-EGFR monoclonal antibodies are being tested as therapy, so that if the patient's cancer is pancreatic cancer or head and neck cancer (in particular squamous cell carcinoma of the head and neck (SCCHN)), then the method according to the invention may be used to predict response either to tyrosine kinase EGFR inhibitors (such as Erlotinib, Gefitinib, or Lapatinib) or to anti-EGFR monoclonal antibodies (such as Cetuximab or Panitumumab).

Cetuximab and Panitumumab are currently the clinically mostly used anti-EGFR monoclonal antibodies. However, further anti-EGFR monoclonal antibodies are in development, such as Nimotuzumab (TheraCIM-h-R3), Matuzumab (EMD 72000), Zalutumumab (HuMax-EGFr), Nimotuzumab and Sym 004. The method according to the invention may also be used to predict response to these anti-EGFR monoclonal antibodies or any other anti-EGFR monoclonal antibodies (including fragments) that might be further developed, in particular if the patient is suffering from colorectal cancer (in particular metastatic colorectal cancer), breast cancer, pancreatic cancer or head and neck cancer (in particular squamous cell carcinoma of the head and neck (SCCHN)).

Similarly, Erlotinib, Gefitinib, Lapatinib and Regorafenib are currently the clinically mostly used tyrosine kinase EGFR inhibitors. However, further tyrosine kinase EGFR inhibitors are in development, such as Canertinib (CI-1033),Neratinib (HKI-272), Afatinib (BIBW2992), Dacomitinib (PF299804,PF-00299804), TAK-285, AST-1306, ARRY334543, AG-1478 (Tyrphostin AG-1478), AV-412, OSI-420 (DesmethylErlotinib), AZD8931, AEE788 (NVP-AEE788), Pelitinib (EKB-569), CUDC-101, AG 490, PD153035 HCl, XL647, Ruxolitinib, and BMS-599626 (AC480).The method according to the invention may also be used to predict response to these tyrosine kinase EGFR inhibitors or any other tyrosine kinase EGFR inhibitors that might be further developed, in particular if the patient is suffering from of lung cancer (in particular non-small cell lung cancer, NSCLC), pancreatic cancer, or head and neck cancer (in particular squamous cell carcinoma of the head and neck (SCCHN)).

### Kits

The present invention also relates to a kit for determining whether a patient with a cancer is likely to respond to an epidermal growth factor receptor (EGFR) inhibitor, comprising or consisting of:
a) reagents for determining the expression level of hsa-miR-31-5p (SEQ ID NO:2) miRNA in a sample (preferably a tumor sample, such as a tumor biopsy or whole or part of a tumor surgical resection) of said patient, and
b) reagents for determining at least one other parameter positively or negatively correlated to response to EGFR inhibitors.

Such reagents may notably include reagents for:
i) determining the expression level of hsa-miR-31-3p (SEQ ID NO:1) miRNA in a sample (preferably a tumor sample, such as a tumor biopsy or whole or part of a tumor surgical resection) of said patient, and/or,
ii) detecting at least one mutation positively or negatively correlated to response to EGFR inhibitors, such as those mentioned in **Table 1** above.

Reagents for determining the expression level of hsa-miR-31-5p or of hsa-miR-31-3p in a sample of said patient, may notably comprise or consist of primers pairs (forward and reverse primers) and/or probes (in particular labeled probes, comprising a nucleic acid specific for the target sequence and a label attached thereto, in particular a fluorescent label) specific for hsa-miR-31-5p and/or has-miR-3p or a microarray comprising a sequence specific for hsa-miR-31-5p and/or hsa-miR-31-3p. The design of primers and/or probe can be easily made by those skilled in the art based on the sequences of hsa-miR-31-5p and/or hsa-miR-31-3p disclosed above.

Reagents for detecting at least one mutation positively or negatively correlated to response to EGFR inhibitors may include at least one primer pair for amplifying whole or part of the gene of interest before sequencing or a set of specific probes labeled with reporter dyes at their 5' end, for use in an allelic discrimination assay, for instance on an ABI 7900HT Sequence Detection System (Applied Biosystems, Foster City, CA) (see Laurent-Puig P, et al, J ClinOncol. 2009, 27(35):5924-30 and Lièvre et al. J ClinOncol. 2008 Jan 20;26(3):374-9 for detection of BRAF mutation V600).

The kit of the invention may further comprise instructions for determining whether the patient is likely to respond to the EGFR inhibitor based on the expression level of hsa-miR-31-5p and the other tested parameter. In particular, a nomogram including points scales of all variables included in the composite score and correlation between the composite score (total number of points) and the prediction (response/non-response or probability of response or non-response) may be included.

### Drugs, therapeutic uses and methods of treating

The method of the invention predicts patient responsiveness to EGFR inhibitors at rates that match reported clinical response rates for the EGFR inhibitors.

It is thus further provided a method for treating a patient with a cancer, which method comprises administering the patient with at least one EGFR inhibitor, wherein the patient has been classified as "responder" or "likely to respond" by the method as described above.

In particular, the invention concerns a method for treating a patient affected with a cancer, which method comprises (i) determining whether the patient is likely to respond to an EGFR inhibitor, by the method according to the invention, and (ii) administering an EGFR inhibitor to said patient if the patient has been determined to be likely to respond to the EGFR inhibitor.

The method may further comprise, if the patient has been determined to be unlikely to respond to the EGFR inhibitor a step (iii) of administering an alternative anticancer treatment to the patient. Such alternative anticancer treatment depends on the specific cancer and on previously tested treatments, but may notably be selected from radiotherapy, other chemotherapeutic molecules, or other biologics such as monoclonal antibodies directed to other antigens (anti-Her2, anti-VEGF, anti-EPCAM, anti-CTLA4...).

In particular, in the case of colorectal cancer, if the patient has been determined to be unlikely to respond to the EGFR inhibitor, the alternative anticancer treatment administered in step (iii) may be selected from:
- a VEGF inhibitor, in particular an anti-VEGF monoclonal antibodies (such as bevacizumab), advantageously in combination with oxaliplatin based chemotherapy such as FOLFOX (a combination of leucovorin (folinic acid), 5-fluorouracil (5-FU), and oxaliplatin) or irinotecan based chemotherapy such as FOLFIRI (a combination of leucovorin (folinic acid), 5-fluorouracil (5-FU), and irinotecan).
- Alternatively, if the patient has already been treated unsuccessfully with a VEGF inhibitor, optionally in combination with axaliplatin or irinotecan based chemotherapy , it may be administered with 5-FU, optionally in combination with Mitomycin B. Best supportive care, defined as a treatment administered with the intent to maximize quality of life without a specific antineoplastic regimen (i.e. not an anticancer treatment) may further be administered to the patient.

Another subject of the invention is an EGFR inhibitor, for use in treating a patient affected with a cancer, wherein the patient has been classified as being likely to respond, by the method as defined above. Said patient may be affected with a colorectal cancer, more particularly a metastatic colorectal cancer. Alternatively, said patient may be affected with a breast cancer, in particular a triple negative breast cancer. Alternatively, said patient may be affected with a lung cancer, in particular a non-small cell lung cancer (NSCLC). Alternatively, said patient may be affected with a head and neck cancer, in particular a squamous-cell carcinoma of the head and neck. Alternatively, said patient may be affected with a pancreatic cancer. The invention also relates to the use of an EGFR inhibitor for the preparation of a medicament intended for use in the treatment of cancer in patients that have been classified as "responder" by the method of the invention as described above.

In a preferred embodiment the EGFR inhibitor is an anti-EGFR antibody, preferably cetuximab or panitumumab. Alternatively, the EGFR inhibitor may be a tyrosine kinase EGFR inhibitor, in particular Erlotinib, Gefitinib, or Lapatinib.

In preferred embodiments:
- the patient is afflicted with a colorectal cancer, in particular a metastatic colorectal cancer, and the EGFR inhibitor is an anti-EGFR antibody, preferably cetuximab or panitumumab;
- the patient is afflicted with a breast cancer, in particular a triple negative breast cancer, and the EGFR inhibitor is an anti-EGFR antibody, preferably cetuximab or panitumumab;
- the patient is afflicted with a lung cancer, in particular a non-small cell lung cancer (NSCLC), and the EGFR inhibitor is a tyrosine kinase EGFR inhibitor, in particular Erlotinib, Gefitinib, or Lapatinib;
- the patient is afflicted with a head and neck cancer, in particular a squamous-cell carcinoma of the head and neck, or a pancreatic cancer, and the EGFR inhibitor is an anti-EGFR antibody (preferably cetuximab or panitumumab) or a tyrosine kinase EGFR inhibitor (in particular Erlotinib, Gefitinib, or Lapatinib).

The examples and figures illustrate the invention without limiting its scope.

### EXAMPLES

### Example 1: Levels of hsa-miR-31-5p in KRAS-wild-type colorectal cancers determine survival differences in patients treated with anti-EGFR

### PATIENTS AND METHODS

### Set of patients

The set of patients was composed of 23 patients with advanced colorectal cancer, all treated with an anti-EGFR antibody after at least 1 line of chemotherapy-based treatment. Eight patients received panitumumab and 14 received cetuximab. One patient received cetuximab and panitumumab. For each patients, formalin fixed, paraffin embedded (FFPE) primary tumor was available. All patients were wild type (WT) for KRAS.

### hsa-miR-31-5p expression analyses:

For each tumor sample, 5µm FFPE slides were scratched in the tumor area and total RNAs were extracted using the FFPE miRNeasy extraction kit (Qiagen, Hilden, Germany) according to the manufacturer's instructions.

Specific quantification of expression level of miRNA hsa-miR-31-5p was performed using specific TaqMan pre-designed assays on retrotranscribed RNA and a ABI7900HT Real-Time PCR System. Expression levels were normalized to the reference through the ΔΔCt method.

### Survival model prediction

A microRNA expression-based predictor of survival risk group was calculated by combining a Cox proportional hazards model (Cox, D. R. (1972). Regression models and life-tables. Journal of the Royal Statistical Society, Series B 34 (2), 187-220) and a supervised principal component method (E Bair & R Tibshirani, Semi-supervised methods to predict patient survival from gene expression data, PLOS Biology 2:511-522, 2004).

A composite prognostic score was calculated for a patient whose expression profile is described by a vector x of log expression levels combining the components of x with the weighted average of each principal component value. A high value of the prognostic score corresponds to a high value of hazard of death, and consequently a relatively poor predicted survival. In order to evaluate the predictive value, leave-one-out cross-validation is used. The score threshold that produced optimal separation between good and bad prognosis was used for Kaplan-Meier analysis.

### RESULTS

Real-time miRNA quantitative PCR analysis using specific TaqMan pre-designed assays was performed on the 23 samples in order to quantify the expression levels of the miRNA hsa-miR-31-5p. Coefficients of the fitted Cox proportional hazards model using the principal components from the dataset were estimated to be 2.6 for PFS (progression free survival). A sample was predicted to be at high/low risk if its prognostic score was larger than/smaller than or equal to 0 (PFS). Patients with high level of hsa-miR-31-5p had a significant shorter PFS than patients with low level of hsa-miR-31-5p with a median PFS of 56.9 weeks for low risk patients and 12.1 weeks for high risk patients (p=0.06) (Figure 1). The prognostic score can then be computed by the following formulae:
PFS score = 0.096* x +0.144, wherein x is the log2 expression of hsa-miR-31-5p.

In their study, Mosakhani et al (Mosakhani N et al. Cancer Genet. 2012 Nov;205(11):545-51) did not identify hsa-miR-31-5p as a predictive marker for response to anti-EGFR antibodies. In the present study, identification of hsa-miR-31-5p as a predictive marker was done considering the whole population together, and with progression free survival as endpoint. In Mosakhani et al. stratification of the population was different. They first divided the population into two groups, a disease control group in which they included responder patients and stable disease patients and a progressive group. They then performed analyses comparing these two groups.

### BIBLIOGRAPHIC REFERENCES

Albitar L et al. Mol Cancer 2010;9:166;
Ambros V et al, RNA 2003 9(3):277-279 ;
Bair E & R Tibshirani, Semi-supervised methods to predict patient survival from gene expression data, PLOS Biology 2:511-522, 2004;
Bhatnagar N et al. Cell Death Dis. 2010 Dec 9;1:e105 ;
Bos. Cancer Res 1989;49:4682-4689;
Bustin et al., 2005, Clin. Sci., 109:365-379;
Chan SL et al. Expert OpinTher Targets. 2012 Mar;16 Suppl 1:S63-8;
Chu H et al. Mutagenesis.2012 Oct 15;
Ciardello F et al. N Engl J Med. 2008 Mar 13;358(11): 1160-74;
Cox, D. R. (1972). Regression models and life-tables. Journal of the Royal Statistical Society, Series B 34 (2), 187-220;
Cunningham et al, N Engl Med 2004;351: 337-45;
Demiralay et al. Surgical Science, 2012, 3, 111-115;
Edkins et al. Cancer BiolTher. 2006 August; 5(8): 928-932;
Geiss GK et al. Nat Biotechnol. 2008 Mar;26(3):317-25 ;
Eisenhauer et al, European Journal of Cancer, 2009, 45:228-247;
Griffiths-Jones S et al. NAR 2006 34(Database Issue):D140-D144;
Griffiths-Jones S et al. NAR 2008 36(Database Issue):D154-D158;
Griffiths-Jones S. NAR 2004 32(Database Issue):D109-D111;
Guo L et al. PLoS One. 2010 Jun 30;5(6):e11387 ;
Kozomara A et al. NAR 2011 39(Database Issue):D152-D157;
Laurent-Puig P, et al, J ClinOncol. 2009, 27(35):5924-30 ;
Laurila EM et al. Genes Chromosomes Cancer. 2013 Dec;52(12):1103-13;
Leboulleux S et al. Lancet Oncol. 2012 Sep;13(9):897-905;
Leslie KK et al. GynecolOncol. 2012 Nov;127(2):345-50;
Li Y et al. Oncol Rep. 2010 Oct;24(4):1019-28;
Liebner DA et al. TherAdvEndocrinolMetab. 2011 Oct;2(5): 173-95;
Lievre et al, Cancer Res. 2006 66(8):3992-5;
Lièvre et al. J ClinOncol. 2008 Jan 20;26(3):374-9;
Liu N et al. Cell Res. 2008 Oct;18(10):985-96;
Mimeault M et al. PLoS One.2012;7(2):e31919;
Mosakhani N et al. Cancer Genet. 2012 Nov;205(11):545-51 ;
Ogino S, et al. J MolDiagn 2008;7:413-21;
Okamura K et al. Nat Struct Mol Biol. 2008 Apr;15(4):354-63;
Pan J et al. Head Neck. 2012 Sep 13;
Ragusa M. et al. Mol Cancer Ther. 2010 Dec;9(12):3396-409;
Shepherd F A, et al, N Engl J Med 2005; 353:123-132;
Tam et al. Clin Cancer Res2006;12:1647-1653 ;
Thomasson M et al. BMC Res Notes.2012 May 3;5:216;
Thomasson M et al. Br J Cancer 2003, 89:1285-1289;
U.S. Pat. No. 7,101,663;
Wang S et al. Tumour Biol. 2014 Aug 20;
Wheeler DL et al. Nat RevClinOncol. 2010 September; 7(9): 493-507 ;
WO2009/080437;
WO2010/121238;
WO2011/135459;
WO2013/076282;
Zeineldin R et al. J Oncol. 2010;2010:414676.

SEQUENCE LISTING

<110>  INTEGRAGEN

<120>  A method for predicting responsiveness to a treatment with an
       EGFR inhibitor

<130>  71558D34198

<160>  12

<170>  PatentIn version 3.5

<210>  1
<211>  22
<212>  RNA
<213>  homo sapiens

<400>  1
ugcuaugcca acauauugcc au                                                22


<210>  2
<211>  21
<212>  RNA
<213>  homo sapiens

<400>  2
aggcaagaug cuggcauagc u                                                 21


<210>  3
<211>  188
<212>  PRT
<213>  homo sapiens

<400>  3

<210> 4
<211> 766
<212> PRT
<213> homo sapiens

<400> 4

<210> 5
<211> 189
<212> PRT
<213> homo sapiens

<400> 5

Met Thr Glu Tyr Lys Leu Val Val Val Gly Ala Gly Gly Val Gly Lys
1               5                   10                  15

Ser Ala Leu Thr Ile Gln Leu Ile Gln Asn His Phe Val Asp Glu Tyr
            20                  25                  30

Asp Pro Thr Ile Glu Asp Ser Tyr Arg Lys Gln Val Val Ile Asp Gly
        35                  40                  45

Glu Thr Cys Leu Leu Asp Ile Leu Asp Thr Ala Gly Gln Glu Glu Tyr
    50                  55                  60

Ser Ala Met Arg Asp Gln Tyr Met Arg Thr Gly Glu Gly Phe Leu Cys
65                  70                  75                  80

Val Phe Ala Ile Asn Asn Ser Lys Ser Phe Ala Asp Ile Asn Leu Tyr
                85                  90                  95

Arg Glu Gln Ile Lys Arg Val Lys Asp Ser Asp Asp Val Pro Met Val
                100                 105                 110

Leu Val Gly Asn Lys Cys Asp Leu Pro Thr Arg Thr Val Asp Thr Lys
            115                 120                 125

Gln Ala His Glu Leu Ala Lys Ser Tyr Gly Ile Pro Phe Ile Glu Thr
    130                 135                 140

Ser Ala Lys Thr Arg Gln Gly Val Glu Asp Ala Phe Tyr Thr Leu Val
145                 150                 155                 160

Arg Glu Ile Arg Gln Tyr Arg Met Lys Lys Leu Asn Ser Ser Asp Asp
                165                 170                 175

Gly Thr Gln Gly Cys Met Gly Leu Pro Cys Val Val Met
                180                 185

<210>  6
<211>  1068
<212>  PRT
<213>  homo sapiens

<400>  6

<210> 7
<211> 1210
<212> PRT
<213> homo sapiens

<400> 7

<210> 8
<211> 705
<212> PRT
<213> homo sapiens

<400> 8

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
                165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620

Thr Tyr Gly Pro Gly Asn Glu Ser Leu Lys Ala Met Leu Phe Cys Leu
625                 630                 635                 640

Phe Lys Leu Ser Ser Cys Asn Gln Ser Asn Asp Gly Ser Val Ser His
                645                 650                 655

Gln Ser Gly Ser Pro Ala Ala Gln Glu Ser Cys Leu Gly Trp Ile Pro
            660                 665                 670

Ser Leu Leu Pro Ser Glu Phe Gln Leu Gly Trp Gly Gly Cys Ser His
        675                 680                 685

Leu His Ala Trp Pro Ser Ala Ser Val Ile Ile Thr Ala Ser Ser Cys
    690                 695                 700

His
705

<210>  9
<211>  628
<212>  PRT
<213>  homo sapiens

<400>  9

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
                165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His

290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620

Thr Tyr Gly Ser
625

<210>  10
<211>  480
<212>  PRT
<213>  homo sapiens

<400>  10

<210>  11
<211>  480
<212>  PRT
<213>  homo sapiens

<400>  11

<210>  12
<211>  480
<212>  PRT
<213>  homo sapiens

<400>  12

## Claims

1. An *in vitro* method for predicting whether a patient with a cancer is likely to respond to an epidermal growth factor receptor (EGFR) inhibitor, which method comprises determining the expression level of hsa-miR-31-5p (SEQ ID NO:2) miRNA in a sample of said patient.

2. The method of claim 1, wherein the patient has a KRAS wild-type cancer.

3. The method of any of claims 1 or 2, wherein the patient is afflicted with a cancer selected from colorectal, lung, breast, ovarian, endometrial, thyroid, nasopharynx, prostate, head and neck, liver, kidney, pancreas, bladder, and brain.

4. The method of claim 3, wherein the cancer is a colorectal cancer, in particular a metastatic colorectal cancer.

5. The method of any of claims 1 to 4, wherein the EGFR inhibitor is an anti-EGFR antibody, in particular cetuximab or panitumumab.

6. The method of any of claims 1 to 5, wherein the sample is a tumor tissue biopsy or whole or part of a tumor surgical resection.

7. The method of any of claims 1 to 6, wherein the level of expression of the miRNA is determined by quantitative RT-PCR.

8. The method of any of claims 1 to 7, wherein the lower the level of expression of the miRNA is, the more likely the patient is to respond to the EGFR inhibitor treatment.

9. The method of any of claims 1 to 8, further comprising determining a prognostic score based on the expression level of the miRNA, wherein the prognostic score indicates whether the patient is likely to respond to the EGFR inhibitor.

10. The method of claim 9, wherein the prognostic score is of formula:
Prognosis score = a * x + b,
wherein:
• x is the logged expression level of hsa-miR-31-5p measured in the patient's sample,
• a and b are parameters that have been previously determined based on a pool of reference samples, and
• the patient is predicted as responding to the EGFR inhibitor if his/her prognosis score is lower than or equal to a threshold value c, and not responding to the EGFR inhibitor if its prognosis score is greater than threshold value c, wherein the value of c has been determined based on the same pool of reference samples.

11. The method of claim 10, wherein a, b and c are preferably in the following ranges:
• a : [0.096], preferably [0.001; 0.12];
• b : [0.144], preferably [0.01; 0.3];and
• c : [-0.1; 0.1], preferably [-0.055; 0.055].

12. The method of any of claims 1 to 9, further comprising determining at least one other parameter positively or negatively correlated to response to EGFR inhibitors, and calculating a composite score taking into account the expression level of hsa-miR-31-5p and said other parameter(s), wherein the composite score indicates whether the patient is likely to respond to the EGFR inhibitor.

13. The method of any of claims 1 to 8, wherein the patient is predicted as likely or unlikely to respond to an EGFR inhibitor based on comparison of the hsa-miR-31-5p expression level in the patient's sample with one or more threshold value(s).

14. A kit for determining whether a patient with a cancer is likely to respond to an epidermal growth factor receptor (EGFR) inhibitor, comprising or consisting of:
a) reagents for determining the expression level of hsa-miR-31-5p (SEQ ID NO:2) miRNA in a sample of said patient, and
b) reagents for determining at least one other parameter positively or negatively correlated to response to EGFR inhibitors.

15. An EGFR inhibitor for use in treating a patient affected with a cancer, wherein the patient has been classified as being likely to respond, by the method according to any of claims 1 to 13.
